Europäisches Patentamt

European Patent Office

Office européen des brevets

(19)

(11) EP 1 093 478 B1

(12) EUROPEAN PATENT SPECIFICATION

(45) Date of publication and mention
of the grant of the patent:
**29.12.2004   Bulletin 2004/53**

(51) Int Cl.7: **C08F 220/04**, A61K 7/00

(21) Application number: **99930250.8**

(86) International application number:
**PCT/US1999/013406**

(22) Date of filing: **14.06.1999**

(87) International publication number:
**WO 1999/065958 (23.12.1999 Gazette 1999/51)**

(54) **ALKALI SOLUBLE LATEX THICKENERS**

IN ALKALI LÖSLICHE VERDICKUNGSMITTEL FÜR LATEX

EPAISSISSANT AU LATEX SOLUBLE AUX ALCALIS

(84) Designated Contracting States:
**AT BE CH DE DK ES FR GB GR IT LI MC NL PT SE**

(30) Priority:  **15.06.1998  US 89216 P**

(43) Date of publication of application:
**25.04.2001   Bulletin 2001/17**

(73) Proprietor: **UNION CARBIDE CHEMICALS &
PLASTICS TECHNOLOGY CORPORATION**
**Danbury, Connecticut 06817-0001 (US)**

(72) Inventors:
 • **BASSETT, David, Robinson
Cary, NC 27513 (US)**
 • **OLESEN, Keith, Russell
Morrisville, NC 27560 (US)**

(74) Representative: **Hayes, Adrian Chetwynd et al
Boult Wade Tennant,
Verulam Gardens
70 Gray's Inn Road
London WC1X 8BT (GB)**

(56) References cited:
EP-A- 0 350 414          WO-A-95/00565
DE-A- 3 735 141          US-A- 4 514 552
US-A- 4 916 183          US-A- 5 382 641

EP 1 093 478 B1

**Description**

**Field of the Invention**

The present invention relates to latex polymers which are alkali soluble and more specifically to latex polymers which demonstrate viscosity enhancement at a pH of less than about 6.

**Background of the Invention**

As used herein, the term "alkali soluble thickeners" means latex polymers which become soluble in aqueous medium upon the addition of a sufficient amount of a base to raise the pH to a level effective to cause the latex polymer to become soluble in the aqueous medium.

Alkali soluble thickeners have found great acceptance in industrial applications, such as, for example, use in coating compositions, e.g., latex paints. Such alkali soluble thickeners are disclosed for example in U.S. Patent Nos. 4,514,552, issued April 30, 1985; 4,722,962 issued February 2, 1988 and 5,292,828 issued march 8, 1994. Such alkali soluble thickeners typically comprise the aqueous emulsion reaction product of (A) a monoethylenically unsaturated carboxylic acid, e.g., methacrylic acid, (B) a monoethylenically unsaturated monomer different from monomer (A), e. g. ethyl acrylate and (C) a macromonomer comprising a hydrophobic portion and an alkoxylated portion which is polymerizable with monomer (A) and monomer (B ). Often, the macromonomer is a urethane monomer which is the urethane reaction product of a monohydric surfactant and a monoethylenically unsaturated monoisocyanate. Typically, the monohydric surfactant comprises an ethyloxated or propoxylated aliphatic alcohol or alkyl phenol.

Typically, alkali soluble thickeners such as described above begin to solubilize and demonstrate viscosity enhancement at a pH of greater than 6.0 and often in the range of about 6.5 or higher. The maximum viscosity is often not achieved until a pH of 7.5 or higher is reached. Accordingly, such alkali soluble thickeners have not found wide acceptance in personal care applications, e.g., hair care and skin care compositions, since personal care compositions typically require significant viscosity enhancement at or below the pH of skin, e.g. from about 6.5 to 6.8.

Accordingly, improved alkali soluble thickeners are desired which can demonstrate viscosity enhancement at a pH of less than about 6.0.

**Summary of the Invention**

By the present invention, it is now possible to provide alkali soluble latex polymers which are suitable for use in personal care applications, e.g., hair care compositions and skin care compositions. The polymers of the present invention comprise the reaction product of (A) an unsaturated carboxylic acid monomer, (B) a monoethylenically unsaturated monomer different from (A), and (C) a macromonomer comprising a hydrophobic portion and an alkoxylated portion which is polymerizable with monomer (A) and monomer (B). Quite surprisingly, in accordance with the present invention, it has been found that when the monoethylenically unsaturated monomer (B) comprises a methyl group, preferably methyl acrylate, solubilization of the polymer occurs at a pH of from about 4.5 to 6, thereby making the polymer suitable for use in personal care applications. In addition, it has been found quite surprisingly in accordance with the present invention that the utilization of a long chain aliphatic alcohol in the preparation of the macromonomer can yield a polymer having desirable shear thinning properties.

**Detailed Description of the Invention**

The unsaturated carboxylic acid monomers suitable for use in accordance with the present invention are typically α,β-monethylenically unsaturated carboxylic acids. Preferred carboxylic acid monomers are selected from the group consisting of acrylic acid, methacrylic acid, crotonic acid, itaconic acid, maleic acid, and mixtures thereof. Methacrylic acid is especially preferred. The concentration of the carboxylic acid monomer is typically from about 20 to 70 weight percent, preferably from about 20 to 50 weight percent and more preferably from about 35 to 45 weight percent based on the total weight of the polymer. The amount of the carboxylic acid monomer is preferably sufficient to provide a polymeric structure which will solubilize and provide viscosity enhancement when reacted with an alkali such as for example, sodium hydroxide.

In accordance with the present invention, the monoethylenically unsaturated monomer different from monomer (A) is one which comprises a methyl group. Preferably, this monomer is an acrylate. More preferably, this monomer is methyl acrylate. Typically, the amount of the monoethylenically unsaturated monomer different from monomer (A) is from about 20 to 80 weight percent, preferably from about 30 to 65 weight percent and more preferably from about 45 to 55 weight percent based on the total weight of the polymer.

The macromonomers suitable for use in accordance with the present invention comprise a hydrophobic portion, an alkoxylated portion and which is polymerizable with monomer (A) and monomer (B). As used herein, the term "macromonomer" means a polymerizable monomer which comprises the reaction product of two or more compounds. Such macromonomers include, for example, any alkoxylated, e.g., ethoxylated or propoxylated, monomers having ethylenic unsaturation and which are terminated by a hydrophobic fatty chain. Examples of unsaturated, polymerizable moieties include those selected from the group consisting of vinyl group containing moieties, methacryloyl, maleoyl, itaconoyl, crotonyl, an unsaturated urethane moiety, hemiester maleoyl, hemiester itaconoyl, $CH_2=CHCH_2O-$, methacrylamido and substituted methacrylamido. Examples of hydrophobic moieties include those selected from the group consisting of alkyl, alkaryl, i.e., alkylaryl or aralkyl, or aryl, linear or branched, saturated or unsaturated, and having at least 6 carbon atoms, preferably from about 6 to 30 carbon atoms per molecule.

Preferred macromonomers are urethane monomers which comprise the reaction product of a monohydric surfactant and a monoethylenically unsaturated isocyanate. Preferably the urethane monomer is a nonionic, urethane monomer which is the urethane reaction product of a monohydric, nonionic surfactant with a monoethylenically unsaturated monoisocyanate, preferably one lacking ester groups, e.g., alpha, alpha-dimethyl-m-isopropenyl benzyl isocyanate. The monohydric nonionic surfactants are themselves well known and are usually alkoxylated, e.g., ethoxylated, hydrophobes containing adducted ethylene oxide to provide the hydrophilic portion of the molecule. The hydrophobes are usually aliphatic alcohols or alkyl phenols in which a carbon chain containing at least 6 carbon atoms, preferably about 6 to 30 carbon atoms, provides the hydrophobic portion of the surfactant. These surfactants are illustrated by ethylene oxide adducts of dodecyl alcohol or octyl or nonyl phenol which are available in commerce and which contain about 5 to about 150, preferably 25 to 60 moles of ethylene oxide per mole of hydrophobe. Other hydrophobic substituents, such as complex hydrophobes, disclosed for example in U.S. Patent 5,488,180 issued January 30, 1996, are suitable for use in accordance with the present invention.

In a preferred aspect of the invention, the hydrophobic portion of the surfactant comprises an aliphatic alcohol containing from about 20 to 24 carbon atoms. More preferably the aliphatic alcohol is of a vegetable origin. Even more preferably, the aliphatic alcohol is behenyl alcohol.

The monoethylenically unsaturated isocyanates suitable for use in preparing the urethane monomers can be any isocyanates effective to form the desired urethane linkage. Preferably, the isocyanate is a monoethylenically unsaturated monoisocyanate. Any copolymerizable unsaturation may be employed, such as acrylate and methacrylate unsaturation. One may also use allylic unsaturation, as provided by allyl alcohol. These, preferably in the form of a hydroxy-functional derivative, as is obtained by reacting a $C_2$-$C_4$ monoepoxide, like ethylene oxide, propylene oxide or butylene oxide, with acrylic or methacrylic acid to form an hydroxy ester, are preferably reacted in equimolar proportions with an organic diisocyanate, such as toluene diisocyanate or isophorone diisocyanate. The preferred monoethylenic monoisocyanate is styryl, as in alpha, alpha-dimethyl-m-isopropenyl benzyl isocyanate, and this unsaturated monoisocyanate lacks the ester group so it forms urethanes which lack this group. The amount of the monoethylenically unsaturated isocyanate relative to the monohydric surfactant used in making the macromonomer, (on a mole ratio basis) is typically from about 0.1-2.0 to 1, preferably about 1.0 to 1.0.

Typically, the amount of monomer (C) is from about 0.5 to 60 weight percent, preferably from about 5 to 50 weight percent and more preferably from about 5 to 15 weight percent based on the total weight of the polymer. Typically, the molecular weight of the macromonomers ranges from about 400 to 8000 grams per gram mole. As used herein, the term "molecular weight" means weight average molecular weight. Techniques for measuring weight average molecular weight are known to those skilled in the art. One such technique, is gel permeation chromatography. Further details concerning the preparation of such macromonomers are known to those skilled in the art and are disclosed for example in U.S. Patent Nos. 4,514,552, 4,801,671, 5,292,828 and 5,294,693.

In addition to the specific monomers described above, additional monomers can be utilized in preparing the polymers of the present invention. For instance, a polyethylenically unsaturated monomer may be copolymerized into the polymer, as is common in alkali soluble emulsion copolymers. These are illustrated by ethylene glycol diacrylate or dimethacrylate 1,6-hexanediol diacrylate or dimethylacrylate, divinyl benzene, and the like. Further details concerning such additional monomers and other monomers suitable for copolymerization in accordance with the present invention are known to those skilled in the art. Such additional monomers typically comprise from 0 to about 10 weight percent based on the total weight of the polymer.

One or more of each class of monomers described above can be used in preparing the polymers of the present invention. Furthermore, in addition to the specific monomers within each class, those skilled in the art will recognize that other monomers within such classes may be utilized in accordance with the present invention. Such monomers are generally commercially available.

The latex polymers of the present invention are prepared in colloidal form, i.e., aqueous dispersions, and can be prepared by emulsion polymerization in the presence of a chain transfer agent and an initiator. Specific details concerning procedures and conditions for emulsion polymerization are known to those skilled in the art. See, for example, U.S. Patent 5,629,375 issued May 13, 1997. Typically, however, the polymerization is carried out in an aqueous medium at a temperature of from about 35 to 90°C. The pressure is not critical and is dependent upon the nature of the monomers employed.

Chain transfer agents are normally not used in the polymerization. However, if chain transfer agents are employed, they are typically present during the polymerization reaction at a concentration of from about 0.01 to 5 weight percent, preferably from about 0.1 to 1 weight percent based on the total monomer content. Both water-insoluble and water-soluble chain transfer agents can be employed. Illustrative of substantially water-soluble chain transfer agents are alkyl and aryl mercaptans such as butyl mercaptan, mercaptoacetic acid, mercaptoethanol, 3-mercaptol-1,2-propanediol and 2-methyl-2-propanethiol. Illustrative of the substantially water-insoluble chain transfer agents include, for example, t-dodecyl mercaptan, phenyl mercaptan, pentaerythritol tetramercaptopropionate, octyldecyl mercaptan, tetradecyl mercaptan and 2-ethylhexyl-3-mercaptopropionate.

In carrying out the emulsion polymerization an initiator is preferably used at a concentration sufficient to initiate the polymerization reaction. This will typically vary from about 0.01 to 3 weight percent based on the weight of monomers charged. However, the concentration of initiator is preferably from about 0.05 to 2 weight percent and, most preferably, from about 0.1 to 1 weight percent of the monomers charged. The particular concentration used in any instance will depend upon the specific monomer mixture undergoing reaction and the specific initiator employed, which details are known to those skilled in the art. Illustrative of suitable initiators include hydrogen peroxide, peracetic acid, t-butyl hydroperoxide, di-t-butyl hydroperoxide, dibenzoyl peroxide, benzoyl hydroperoxide, 2,4-dicholorbenzoyl peroxide, 2,5-dimethyl-2,5-bis(hydroperoxy) hexane, perbenzoic acid, t-butyl peroxypivalate, t-butyl peracetate, dilauroyl peroxide, dicapryloyl peroxide, distearoyl peroxide, dibenzoyl peroxide, diisopropyl peroxydicarbonate, didecyl peroxydicarbonate, dicicosyl peroxydicarbonate, di-t-butyl perbenzoate, 2,2'-azobis-2,4-dimethylvaleronitrile, ammonium persulfate, potassium persulfate, sodium persulfate, sodium perphosphate, azobisisobutyronitrile, as well as any of the other known initiators. Also useful are the redox catalyst systems such as sodium persulfate-sodium formaldehyde sulfoxylate, t-butyl hydroperoxide - isoascorbic acid, , t-butyl hydroperoxide-sodium persulfate-sodium formaldehyde sulfoxylate, cumene hydroperoxide-sodium metabisulfite, hydrogen peroxide-ascorbic acid, and other known redox systems. Moreover, as known by those skilled in the art, traces of metal ions can be added as activators to improve the rate of polymerization, if desired.

The particular surfactant useful for conducting the polymerization reaction is not critical to the present invention. Typical surfactants include anionic surfactants such as sodium lauryl sulfate, sodium tridecylether sulfate, diester sulfosuccinates and sodium salts of alkyl aryl polyether sulfonates; and nonionic surfactants such as alkyl aryl polyether alcohols and ethylene oxide condensates of propylene oxide, propylene glycol adducts. Preferably, the initiators and surfactants are selected to minimize the odor of the final product.

The reaction products comprising the latex polymers of the present invention typically have a solids, i.e. polymer, content of from about 10 to 65 weight percent, preferably from about 20 to 50 weight percent based on the weight of the latex and water. Typically, the particle size of the latex polymer is from about 0.1 to 1.0 microns. The Tg of the latex polymers of the present invention is typically from about 20 to 60 C. As used herein, the term "Tg" means polymer glass transition temperature. Techniques for measuring the glass transition temperature of polymers are known to those skilled in the art. One such technique is, for example, differential scanning calorimetry. A particularly useful means of estimating the glass transition temperature of a polymer is that given Fox,

$$1/Tg_{(polymer)} = x_1/Tg_1 + x_2/Tg_2 + x_3/Tg_3 + ... + x_n/Tg_n \qquad (1)$$

where $x_i$ is the weight fraction of component i in the copolymer and $Tg_i$ is the homopolymer glass transition of component i. The homopolymer glass transition temperatures can be found in any publicly available source such as the Polymer Handbook.

Typically the viscosity of the latex polymers of the present invention is from about 5 to 1500 centipoise ("cP") in the unneutralized form measured at 20°C with a 20 to 50 weight percent solids composition using a Brookfield Viscometer with a number 2 spindle at 60 revolutions per minute. The molecular weight of the latex polymers of the present invention is typically from about $10^4$ to $10^7$, preferably from about 200,000 to 1,000,000 grams per gram mole.

In accordance with the present invention the latex polymers begin to become soluble in the aqueous medium at a pH of less than about 6.0, preferably at a pH of from about 4.5 to 6.0, and more preferably at a pH from about 5.0 to 6.0. Significant solubilization, i.e., 90 % of maximum viscosity, preferably occurs at a pH of about 7.5 or less, more preferably at a pH of about 7.0 or less. The solubilization is effected by the introduction of a suitable alkali, such as, for example, sodium hydroxide, potassium hydroxide, ammonium hydroxide, amines, for example, triethyl amine, and other alkali materials known to those skilled in the art. Upon the solubilization of the latex polymers, the viscosity of the aqueous medium demonstrates a significant increase. Preferably in accordance with the present invention, aqueous compositions comprising the solubilized polymers of the present invention have a viscosity of greater than about 10,000 cP, preferably greater than about 20,000 cP, more preferably greater than about 40,000 cP, and most preferably greater than about 60,000 cP at a pH of less than about 6.0. Unless otherwise stated, the viscosity measurement of the solubilized polymer is taken at a polymer concentration of 1.0 weight percent based on the total weight of the aqueous composition using a Brookfield LVT DV-II viscometer at 20°C using a No. 3 spindle at 12 rpm.

Quite surprisingly, in accordance with the present invention, it has been found that by utilizing a straight chain aliphatic alcohol having from about 20 to 24 carbon atoms as the hydrophobe in the manufacture of the macromonomer, the polymers of the present invention in their solubilized state have desirable shear thinning characteristics. Note the following data in Table 1 below.

TABLE 1

| Spindle Speed (rpm) | Viscosity, cPs |
|---|---|
| 0.3 | 1658550 |
| 0.6 | 895220 |
| 1.5 | 396200 |
| 3.0 | 213860 |
| 6.0 | 115430 |
| 12.0 | 62300 |
| 30.0 | 27575 |
| 60.0 | 14885 |

In general, the polymers of the present invention are particularly effective as thickeners, i.e., viscosifiers, for aqueous compositions. Typically, the amount of the polymer employed to achieve thickening efficiency, is from about 0.05 to about 5 weight percent based on the total weight of the composition. The specific amount of polymer will be dependent upon the particular end use and can be determined by those skilled in the art.

In addition, other ingredients, such as, for example, surfactants, preservatives, co-thickeners, such as, for example, polysaccharide ethers, solvents and other materials known to those skilled in the art can be employed in the compositions of the present invention.

The polymers described herein are useful in a variety of aqueous systems, such as textile coatings (woven and nonwoven), latex paint formulations, cosmetic formulations, pigment dispersions and slurries, dentrifices, hand lotions, liquid detergents, quenchants, agricultural chemicals, concrete additives, transmission fluids, waste water treatment (flocculants), turbulent drag reduction, automation coatings (OEM and refinish), architectural coatings, industrial coatings and the like. Other applications include, for example, paper making, mineral processing, brine viscosification, superabsorbency, enhanced oil recovery, personal care products, biomedical, pharmaceutical and the like.

The polymers of the present invention are particularly suitable for use in personal care compositions, e.g., hair care compositions and skin care compositions. Typical personal care compositions comprise the polymer of the present invention, water and a suitable personal care ingredient.

As used herein, the term "personal care ingredient" includes, but is not limited to, active ingredients, such as, for example, spermicides, virucides, analgesics, anesthetics, antibiotic agents, antibacterial agents, antiseptic agents, vitamins, corticosteriods, antifungal agents, vasodilators, hormones, antihistamines, autacoids, kerolytic agents, antidiarrhea agents, anti-alopecia agents, anti-inflammatory agents, glaucoma agents, dry-eye compositions, wound healing agents, exfoliating agents, sunscreens, anti-oxidants, enzymes, anti-infection agents, and the like, as well as solvents, diluents and adjuvants such as, for example, water, ethyl alcohol, isopropyl alcohol, higher alcohols, glycerine, propylene glycol, sorbitol, preservatives, surfactants, menthol, eucalyptus oil, other essential oils, fragrances, viscosity adjusters, polymers other than those of the present invention, and the like.

Such other polymers include, for example, naturally occurring and synthetic neutral, cationic, anionic and amphoteric polymers, e.g., polysaccharide ethers, hyaluronic acid, polyalkylene oxides and polycarboxylic acids. The polysaccharides include naturally occurring, biosynthesized and derivatized carbohydrate polymers or mixtures thereof. Such materials encompass high molecular weight polymers composed of monosaccharide units joined by glycosidic bonds. These materials may include, for example, the entire starch and cellulose families; pectin, chitosan; chitin; the seaweed products such as agar and carrageenan; alginate; the natural gums such as guar, arabic and tragacanth; bio-derived gums such as xanthan; and the like. Common polysaccharides include cellulosics conventionally employed for the preparation of cellulose ethers, such as, for example, chemical cotton, cotton linters, wood pulp, alkali cellulose and the like. Such materials are commercially available. The molecular weight of the polysaccharides typically ranges from about 10,000 to 2,000,000 grams per gram mole.

Preferably, the polysaccharides are etherified by reacting the polysaccharide with an alkylene oxide, e.g., ethylene oxide, propylene oxide or butylene oxide. The amount of ether substitution is typically from about 1.5 to 6 and preferably from about 2 to 4 moles of ether substituent per mole of polysaccharide ether. The polysaccharide ethers may be substituted with one or more desired substituents, e.g., cationic, anionic and/or hydrophobic substituents. Hydrophobic substituents are known in the art, examples of which are described above. Hydrophobically-modified cellulose ethers are described, for example, in U.S. Patent Nos. 4,228,277, 5,120,328 and 5,504,123 and European Patent Publication 0 384 167 B1. Cationic, hydrophobically modified cellulose ethers are described, for example, in U. S. Patent No. 4,663,159. The substitution level of each such substituent on the polysaccharide ether is typically from about 0.001 to 0.1 and preferably from about 0.004 to about 0.05 moles of substituent per mole of polysaccharide ether.

Preferred polysaccharide ethers for use in accordance with the present invention, are cellulose ethers, including for example, hydroxyethyl cellulose, hydroxypropyl cellulose, methyl cellulose, ethyl cellulose, hydroxypropyl methyl cellulose, hydroxyethyl methyl cellulose, carboxymethyl cellulose, hydroxyethyl carboxylmethyl cellulose, and derivatives thereof.

Such personal care ingredients are commercially available, e.g., from Amerchol Corporation, Edison, NJ. Further details concerning the selection and amounts of such personal care ingredients are known to those skilled in the art.

Typical personal care compositions in accordance with the present invention may, for example, contain the following ingredients with the proportions listed below as weight percent active. The ingredients are listed as International Nomenclature of Cosmetic Ingredients ("INCI") names which can be found, for example, in the International Cosmetic Industrial Dictionary, 6th Editon, Cosmetic Toiletries and Fragrances Association (1995).

## TYPICAL FORMULATIONS

| Mousse for skin conditioning | |
|---|---|
| Ingredients: | % |
| Oleth-10 | 1.00 |
| PPG-10 Methyl Glucose Ether | 3.00 |
| Deionized Water | q.s. 100 |
| PEG 14M | 0.10 |
| Polymer of Invention | 0.10 |
| Aminomethylpropanol | q.s.pH=5.5 |
| Preservatives | q.s. |
| SD Alcohol 40 | 15.00 |
| A-46 Hydrocarbon propellent | 4.00 |

| Mousse for hair styling/conditioning | |
|---|---|
| Ingredients: | % |
| Dimethicone Copolyol | 1.00 |
| PVP/VA Copolymer | 2.00 |
| Polyquaternium-24 | 0.30 |
| Deionized Water | q.s. 100 |
| Polymer of Invention | 0.125 |
| Aminomethylpropanol | q.s. pH=5.5 |
| SD Alcohol 40 | 15.00 |
| A-46 Hydrocarbon Propellent | 4.00 |

| Styling Gel | |
|---|---|
| Ingredients: | % |
| Deionized Water | q.s. 100 |
| Polymer of Invention | 1.25 |
| PVP/VA Copolymer | 4.00 |

(continued)

| Styling Gel | |
|---|---|
| Ingredients: | % |
| Triethanolamine | q.s. pH=5.8 |
| Methyl Gluceth-20 | 5.00 |
| Preservatives | q.s. |
| Color | q.s. |

| Hand Lotion | |
|---|---|
| Ingredients: | % |
| Mineral Oil | 8.00 |
| Cetyl Acetate/Acetylated Lanolin Alcohol | 1.00 |
| Glyceryl Stearate | 0.50 |
| Methyl Glucose Sesquistearate | 3.00 |
| PEG-20 Methyl-Glucose Sesquistearate | 2.00 |
| Dimethicone | 0.50 |
| Deionized water | q.s. 100 |
| Methyl Gluceth-10 | 0.50 |
| Polymer of Invention | 0.20 |
| Preservatives | q.s. 100 |
| Triethanolamine | q.s. pH = 7.0 |

| Spray Gel | |
|---|---|
| Ingredients: | % |
| Deionized water | q.s. 100 |
| Polymer of Invention | 0.30 |
| Methyl Gluceth-20 | 4.00 |
| Dimethicone Copolyol | 2.00 |
| Triethanolamine | q.s. pH = 5.8 |
| PVP/VA Copolymer | 5.00 |

| Curl Activator | |
|---|---|
| Ingredients: | % |
| Deionized water | q.s. 100 |
| Polymer of Invention | 0.50 |
| Glycerine | 10.00 |
| Propylene Glycol | 5.00 |
| Methyl Gluceth-10 | 5.00 |
| Preservative | q.s. |
| Triethanolamine | q.s. pH = 6.0 |

| Shampoo | |
|---|---|
| Ingredients: | % |
| Deionized water | q.s. 100 |

(continued)

| Shampoo | |
|---|---|
| Ingredients: | % |
| Ammonium Laureth Sulfate | 10.00 |
| Ammonium Lauryl Sulfate | 5.00 |
| Cocamide MEA | 3.00 |
| Coco-Betaine | 1.00 |
| Polymer of Invention | 1.0 |
| Dimethicone | 1.00 |
| Deionized Water | 10.00 |
| Polyquaterinium-10 | 0.30 |
| Triethanolamine | q.s. pH = 6.5 |
| Preservatives | q.s. |

| Cream Rinse | |
|---|---|
| Ingredients: | % |
| Cetearyl Alcohol (and) Ceteareth-20 | 4.00 |
| Cetyl Alcohol | 1.00 |
| Polysorbate 80 | 0.50 |
| Deionized Water | q.s. 100 |
| Polymer of Invention | 0.50 |
| Triethanolamine | q.s. pH =6.0 |
| Lauryl Methyl Gluceth-10 | 1.00 |
| Hydroxypropyldimonium Chloride | |
| Preservatives | q.s. |

The invention is hereinafter described with reference to the Examples which are not intended to limit the claims which follow.

The following Example is illustrative of the preparation of the nonionic urethane monomers of this invention.

EXAMPLE 1

(Preparation of a Urethane Monomer)

To a one-liter glass reactor fitted with a thermometer, heating mantle, thermoregulator, stirrer, nitrogen sparge, and condenser including a Dean-Stark trap is charged 800.0 g of a 40 mole ethoxylate of behenyl alcohol as a hot melt. The reactor contents are heated, with nitrogen sparging, to 110o C. and held for two hours while trace moisture is removed and collected in the Dean-Stark Trap (typically less than 1 g).

The reactor contents are then cooled to 80o C., the Dean Stark trap is replaced with a condenser, and the nitrogen sparge is switched to an air sparge for 15 minutes. With continued air sparging, 0.02 g methoxyhydroquinone inhibitor, 0.50 g dibutyl tin dilaurate catalyst, and 81.0 g of alpha, alpha-dimethyl-m-isopropenyl benzyl isocyanate (m-TMI, a product of Cytec Industries, Inc. Stamford, CT, may be used) are charged in order to the reactor. After a rapid initial exotherm which increases the reaction temperature about 8o C., the contents are heated to maintain 80o C. for an additional two hours. The product is then cooled to room temperature.

The final product is a white wax in appearance with residual isocyanate content of less than 0.5% and with 98% of the original ethylenic unsaturation retained.

EXAMPLE 2

(Preparation of an Alkali-Soluble Thickener with Urethane Monomer)

To a three-liter flask equipped with thermometer, stirrer, condenser, nitrogen inlet, thermoregulated water bath and monomer addition pump is charged 989.0 g deionized water and 4.0 g of 2-sulfoethyl methacrylate. The water is heated to 80o C. and purged with nitrogen for 30 minutes.

A pre-mix of monomers is prepared in a separate stirred container by charging, in order, 171.0 g methacrylic acid, 203.0 g methyl acrylate, 21.0 g dioctyl sodium sulfosuccinate, Triton GR-9M surfactant, a product of Union Carbide Corporation (sodium lauryl sulfate may be used as an alternative) and 56.0 g of the ethoxylated urethane monomer prepared in Example 1.

Under a nitrogen blanket, 45.0 g (10%) of the monomer pre-mix is charged to the reactor followed by 36 g of 1% sodium persulfate, 3% sodium bicarbonate solution. The contents exotherm to about 85o C., and after cooling back to 80o C. The addition of the remaining pre-mix is started and continued progressively over 2.5 hours until complete. The reactor contents are heated for an additional 30 minutes at 80o C. to complete the conversion of monomer to copolymer and then cooled to 60 C, at which point 2.0 g of a 6 wt% solution of t-butyl hydroperoxide is added followed by 2.0 g of a 6 wt% solution of isoascorbic acid added over 15 minutes to scavenge residual monomer to below 2 ppmw.

The product is a low viscosity latex of solids content 25.1%, RVT Brookfield viscosity 19 cps (No. 1 spindle at 100 rpm), pH of 2.5, and average particle size of 92 nanometers ("nm").

Example 3

Control

Preparation of Alkali Soluble Thickener having Ethyl Group with Urethane Monomer

A polymer is prepared in accordance with the procedure set forth in Example 2 with the exception that ethyl acrylate is substituted for the methyl acrylate referred to in Example 2.

The product is a low viscosity latex of solids content 25.0%, RVT Brookfield viscosity 19 cps (No. 1 spindle at 100 rpm), pH of 2.6, and average particle size of 92 nm.

Example 4

Viscosity Dependence on pH

The polymers prepared in Examples 2 and 3 were treated with various amounts of an alkaline material, e.g., 10% NaOH and the viscosity was measured as a function of pH. The viscosity was measured using a Brookfield LVT DV-II Viscometer with a # 3 spindle at a concentration of 1.00 weight percent polymer at 20°C and a speed of 12 rpm.

The results are shown in Table 2 below.

TABLE 2

| pH Dependence | | |
| --- | --- | --- |
| | Example 2 | Example 3 (control) |
| pH | Viscosity, cP | Viscosity, cP |
| 4 | 1 | 1 |
| 4.5 | 1 | 1 |
| 5.0 | 1,000 | 1 |
| 5.5 | 35,000 | 1 |
| 6.0 | 75,000 | 1 |
| 6.5 | 80,000 | 60,000 |
| 7.0 | 85,000 | 80,000 |

It can be seen from the data presented in Table 1 that quite surprisingly, the replacement of ethyl acrylate with methyl acrylate in the polymer of the present invention resulted in a substantial reduction in the pH necessary to achieve viscosification of the aqueous composition.

Although the present invention has been described with respect to specific aspects, those skilled in the art will recognize that other aspects are intended to be included within the scope of the claims which follow.

**Claims**

1. A polymer comprising the reaction product of:

   (A) an unsaturated carboxylic acid monomer;
   (B) a monoethylenically unsaturated monomer different from monomer (A); and
   (C) a macromonomer comprising a hydrophobic portion and an alkoxylated portion which is polymerizable with monomer (A) and monomer (B): **characterized in that**:

      (i) the monoethylenically unsaturated monomer (B) comprises a methyl group; and
      (ii) an aqueous solution of the polymer at a concentration of 1.0 weight percent has a viscosity of at least 10 Pas (10,000 cP) at a pH of less than 6.0.

2. The polymer of claim 1 wherein the hydrophobic macromonomer is a urethane monomer comprising the reaction product of a monohydric surfactant and a monoethylenically unsaturated isocyanate.

3. The polymer of claim 2 wherein the monohydric surfactant is an alkoxylated aliphatic alcohol or alkyl phenol.

4. The polymer of claim 3 in which said monohydric surfactant has the formula:

$$R-O-(CH_2-CHR'O)_m-(CH_2-CH_2O)_n-H$$

   in which R is an alkyl group containing 6 to 30 carbon atoms or an alkaryl group containing 8 to 30 carbon atoms, R' is C1-C4 alkyl, n is an average number from 6 to 150, and m is an average number of from 0 to 50 provided n is at least as great as m and n + m = 6 to 150.

5. The polymer of any one of claims 2 to 4 in which said monomer (C) is a urethane reaction product of said monohydric surfactant with alpha, alpha-dimethyl-m-isopropenyl benzyl isocyanate.

6. The polymer of any one of the preceding claims wherein monomer (B) is an acrylate.

7. The polymer of claim 6 wherein monomer (B) is methyl acrylate.

8. The polymer of any one of the preceding claims which comprises from 20 to 80 weight percent of monomer (B) based on the total weight of the polymer.

9. The polymer of any one of the preceding claims wherein the unsaturated carboxylic acid monomer is selected from the group consisting of acrylic acid, methacrylic acid, crotonic acid, itaconic acid and mixtures thereof.

10. The polymer of any one of the preceding claims which comprises from 20 to 70 weight percent of monomer (A) based on the total weight of the polymer.

11. The polymer of any one of the preceding claims wherein the hydrophobic portion of the macromonomer is an aliphatic alcohol of a vegetable origin.

12. The polymer of claim 11 wherein the alcohol has from 20 to 24 carbon atoms.

13. The polymer of claim 12 wherein the alcohol is behenyl alcohol.

14. The polymer of any one of the preceding claims which comprises from 0.5 to 60 weight percent of monomer (C) based on the total weight of the polymer.

15. The polymer of any one of the preceding claims comprising 35 to 45 weight percent of monomer (A), from 45 to 55 weight percent of monomer (B) and from 5 to 15 weight percent of monomer (C) based on the total weight of the polymer.

16. A composition comprising the polymer of any one of the preceding claims and water.

**17.** The composition of claim 16 further comprising at least one personal care ingredient.

**18.** The composition of claim 16 or 17 having a viscosity of at least 20 Pas (20,000 cP) at a pH of less than 6.0.

**Patentansprüche**

**1.** Polymer mit dem Reaktionsprodukt von:

    (A) einem ungesättigten Carbonsäuremonomer;
    (B) einem monoethylenisch ungesättigten Monomer, das von Monomer (A) verschieden ist; und
    (C) einem Makromonomer mit einem hydrophoben Teil und einem alkoxylierten Teil, das mit Monomer (A) und Momomer (B) polymerisierbar ist;

**dadurch gekennzeichnet, daß**:

    (i) das monoethylenisch ungesättigte Monomer (B) eine Methylgruppe umfaßt; und
    (ii) eine wäßrige Lösung des Polymers bei einer Konzentration von 1,0 Gew.-% eine Viskosität von mindestens 10 Pas (10.000 cP) bei einem pH-Wert von weniger als 6,0 hat.

**2.** Polymer nach Anspruch 1, bei dem das hydrophobe Makromonomer ein Urethanmonomer mit dem Reaktionsprodukt eines einwertigen Tensids und eines monoethylenisch ungesättigten Isocyanats ist.

**3.** Polymer nach Anspruch 2; bei dem das einwertige Tensid ein alkoxylierter aliphatischer Alkohol oder Alkylphenol ist.

**4.** Polymer nach Anspruch 3, bei dem das einwertige Tensid die folgende Formel hat:

$$R-O-(CH_2-CHR'O)_m-(CH_2-CH_2O)_n-H$$

bei der R eine Alkylgruppe ist, die 6 bis 30 Kohlenstoffatome enthält, oder eine Alkylarylgruppe, die 8 bis 30 Kohlenstoffatome enthält, R' $C_1$-$C_4$-Alkyl ist, n eine gemittelte Zahl von 6 bis 150 ist und m eine gemittelte Zahl von 0 bis 50 ist, vorausgesetzt n ist mindestens so groß wie m und n + m = 6 bis 150.

**5.** Polymer nach einem der Ansprüche 2 bis 4, bei dem das Monomer (C) ein Urethanreaktionsprodukt des einwertigen Tensids mit $\alpha,\alpha$-Dimethyl-misopropenylbenzylisocyanat ist.

**6.** Polymer nach einem der vorhergehenden Ansprüche, bei dem Monomer (B) ein Acrylat ist.

**7.** Polymer nach Anspruch 6, bei dem Monomer (B) Methylacrylat ist.

**8.** Polymer nach einem der vorhergehenden Ansprüche, das 20 bis 80 Gew.-% Monomer (B) bezogen auf das Gesamtgewicht des Polymers umfaßt.

**9.** Polymer nach einem der vorhergehenden Ansprüche, bei dem das ungesättigte Carbonsäuremonomer ausgewählt ist aus der aus Acrylsäure, Methacrylsäure, Crotonsäure, Itaconsäure und Mischungen derselben bestehenden Gruppe.

**10.** Polymer nach einem der vorhergehenden Ansprüche, das 20 bis 70 Gew.-% Monomer (A) bezogen auf das Gesamtgewicht des Polymers umfaßt.

**11.** Polymer nach einem der vorhergehenden Ansprüche, bei dem der hydrophobe Teil des Makromonomers ein aliphatischer Alkohol von pflanzlichem Ursprung ist.

**12.** Polymer nach Anspruch 11, bei dem der Alkohol 20 bis 24 Kohlenstoffatome hat.

**13.** Polymer nach Anspruch 12, bei dem der Alkohol Behenylalkohol ist.

**14.** Polymer nach einem der vorhergehenden Ansprüche, das 0,5 bis 60 Gew.-% Monomer (C) bezogen auf das Gesamtgewicht des Polymers umfaßt.

**15.** Polymer nach einem der vorhergehenden Ansprüche mit 35 bis 45 Gew.-% Monomer (A), 45 bis 55 Gew.-% Monomer (B) und 5 bis 15 Gew.-% Monomer (C) bezogen auf das Gesamtgewicht des Polymers.

**16.** Zusammensetzung, die das Polymer nach einem der vorgehenden Ansprüche und Wasser umfaßt.

**17.** Zusammensetzung nach Anspruch 16, die ferner mindestens einen Körperpflegebestandteil umfaßt.

**18.** Zusammensetzung nach Anspruch 16 oder 17 mit einer Viskosität von mindestens 20 Pas (20.000 cP) bei einem pH-Wert von weniger als 6,0.

**Revendications**

**1.** Polymère comprenant le produit de réaction :

A) d'un monomère acide carboxylique insaturé,
B) d'un monomère à insaturation monoéthylénique, différent du monomère (A),
C) et d'un macromonomère comportant un fragment hydrophobe et un fragment alcoxylé, qui peut polymériser avec les monomères (A) et (B),

et **caractérisé en ce que**

i) le monomère (B) à insaturation monoéthylénique comporte un groupe méthyle,
ii) et une solution aqueuse à 1,0 % en poids du polymère présente, à un pH inférieur à 6,0, une viscosité d'au moins 10 Pa.s (10 000 cP).

**2.** Polymère conforme à la revendication 1, dans lequel le macromonomère hydrophobe est un monomère uréthane constitué par le produit de réaction d'un tensioactif à un seul atome d'hydrogène actif et d'un isocyanate à insaturation monoéthylénique.

**3.** Polymère conforme à la revendication 2, dans lequel le tensioactif à un seul atome d'hydrogène actif est un produit d'alcoxylation d'un alcool aliphatique ou d'un alkyl-phénol.

**4.** Polymère conforme à la revendication 2, dans lequel le tensioactif à un seul atome d'hydrogène actif a pour formule

$$R-O-(CH_2-CHR'-O)_m-(CH_2-CH_2-O)_n-H$$

dans laquelle R représente un groupe alkyle comportant 6 à 30 atomes de carbone ou un groupe alkyl-aryle comportant 8 à 30 atomes de carbone, R' représente un groupe alkyle en $C_{1-4}$, n est un nombre qui vaut en moyenne de 6 à 150, et m est un nombre qui vaut en moyenne de 0 à 50, sous réserve que n soit au moins égal à m et que la somme n + m vaille de 6 à 150.

**5.** Polymère conforme à l'une des revendications 2 à 4, dans lequel ledit monomère (C) est un uréthane qui est le produit de réaction dudit tensioactif à un seul atome d'hydrogène actif et de l'isocyanate d'$\alpha,\alpha$-diméthyl-m-isoprényl-benzyle.

**6.** Polymère conforme à l'une des revendications précédentes, dans lequel ledit monomère (B) est un acrylate.

**7.** Polymère conforme à la revendication 6, dans lequel le monomère (B) est l'acrylate de méthyle.

**8.** Polymère conforme à l'une des revendications précédentes, dans lequel le monomère (B) représente de 20 à 80 % du poids total du polymère.

**9.** Polymère conforme à l'une des revendications précédentes, dans lequel le monomère acide carboxylique insaturé est choisi dans l'ensemble constitué par les acides acrylique, méthacrylique, crotonique et itaconique, ainsi que leurs mélanges.

**10.** Polymère conforme à l'une des revendications précédentes, dans lequel le monomère (A) représente de 20 à 70 % du poids total du polymère.

**11.** Polymère conforme à l'une des revendications précédentes, dans lequel le fragment hydrophobe du macromonomère est un alcool aliphatique d'origine végétale.

**12.** Polymère conforme à la revendication 11, dans lequel ledit alcool comporte de 20 à 24 atomes de carbone.

**13.** Polymère conforme à la revendication 12, dans lequel ledit alcool est l'alcool béhénylique.

**14.** Polymère conforme à l'une des revendications précédentes, dans lequel le monomère (C) représente de 0,5 à 60 % du poids total du polymère.

**15.** Polymère conforme à l'une des revendications précédentes, dans lequel les monomères (A), (B) et (C) représentent respectivement de 35 à 45 %, de 45 à 55 % et de 5 à 15 % du poids total du polymère.

**16.** Composition comprenant un polymère conforme à l'une des revendications précédentes et de l'eau.

**17.** Composition conforme à la revendication 16, qui comprend en outre au moins un ingrédient de soins personnels.

**18.** Composition conforme à la revendication 16 ou 17, qui présente, à un pH inférieur à 6,0, une viscosité d'au moins 20 Pa.s (20 000 cP).